# EUROPEAN PATENT SPECIFICATION

(11) **EP 2 782 508 B1**
(45) Date of publication and mention of the grant of the patent: **03.02.2016**
(21) Application number: 12790900.0
(22) Date of filing: 22.11.2012
(51) Int. Cl.: A61B 17/322, A61B 17/3205, A61B 17/34

(54) **DEVICE FOR TISSUES SAMPLING AND GRAFTING**
VORRICHTUNG FÜR GEWEBEABTASTUNG UND -PFROPFUNG
DISPOSITIF D'ÉCHANTILLONNAGE ET DE GREFFE DE TISSUS

(30) Priority: 23.11.2011 IT BO20110667
(43) Date of publication of application: 01.10.2014
(73) Proprietor: Andreani, Silvano, 47893 Borgo Maggiore (SM); Casale, Ettore, 09134 Quartu Sant'Elena (CA) (IT); Maullu, Giorgio, 09170 Oristano (OR) (IT)
(72) Inventor: Andreani, Silvano, 47893 Borgo Maggiore (SM); Casale, Ettore, 09134 Quartu Sant'Elena (CA) (IT); Maullu, Giorgio, 09170 Oristano (OR) (IT)
(74) Representative: Negrini, Elena
(86) International application number: PCT/EP2012/073401
(87) International publication number: WO 2013/076213

(56) References cited:
- EP-A1- 1 312 315
- FR-A1- 2 696 334
- JP-A- 2007 229 330
- US-A1- 2004 193 203

## Description

### Technical Field

The present invention relates to the technical field of surgical devices and particularly relates to a device for tissues sampling and grafting.

In the field of tissue sampling and grafting and especially of those that constitute the follicular units are generally used two specific tools to realize the individual sampling and grafting steps.

The sampling step is performed using an extractor device, similar to a coring drill, consisting of a hole saw, rotated by a micro-motor and connected to a suction unit. Then the hole saw performs a sort of coring of the follicular unit with a part of the surrounding skin tissue which is conveyed by means of a suction unit, generally consisting of a vacuum pump connected through a duct to the device, into a collection container.

The operator performs a series of sampling at the end of which empties the collecting container and evaluates the follicular units suitable for subsequent grafting step.

First the grafting step involves the creation of a series of grafting holes using a drill, then a suction step of a single follicular unit through a needle to be grafted and finally the grafting of this unit within the corresponding follicular grafting hole.

Document JP 2007 229330 discloses a prior art device for tissues sampling and grafting according to the preamble of claim 1.

### Technical Problem

The main disadvantage of the prior art concerns the large number of operations to be performed by the operator to sample and subsequently to graft each follicular unit. Due to the large number of manipulations suffered by each follicular unit, the latter is particularly stressed and "mishandled" so as to significantly reduce the grafting success rate.

Further disadvantage of the prior art concerns the high bleeding of the affected tissues during the grafting step and especially during the graft holes preparation step.

### Technical Solution

The main object of the present invention is to propose a device for tissue sampling and grafting, according to independent claim 1, that reduces the number of operations to be performed also reducing the stress applied to the concerned tissue to be sampled and grafted thereby improving their engraftment chances. Preferred embodiments are disclosed in the dependent claims.

Further object is to simplify and speed up the sampling and grafting operations using a single device.

Another object is to propose a device suitable to the auto transplant operations of epithelial tissues, removing of scars, and epidemic damage due to burns and/or tattoos.

Further object is to propose a device which allows to visually evaluate the taken tissue sample condition before the grafting of the same to ensure a higher grafting success rate.

### Brief Description of Drawings

The characteristics of the invention are highlighted in the following with particular reference to the accompanying drawings wherein:
- Fig. 1 shows a front view of a device according to the present invention;
- Figs. 2 and 3 show a front view of the device of Fig. 1 wherein some portions have been removed to better illustrate others;
- Figs. 4, 5, 6, 8 to 13 show a front view of respective constituent parts of the device of Fig. 1;
- Fig. 7 shows a sectional view of the detail shown in Fig. 6;
- Figs 14 and 16 respectively show the device wherein some portions have been removed to better show others and its own particular in correspondence of a first operating condition;
- Figs. 15 and 17 respectively show the device wherein some portions have been removed to better show others and its own particular in correspondence of a second operating condition;
- Figs. 18 and 20 respectively show the device wherein some portions have been removed to better show others and its own particular in correspondence of a third operating condition;
- Figs. 19 and 21 respectively show the device wherein some portions have been removed to better show others and its own particular in correspondence of a fourth operating condition;
- Fig. 22 shows a top view of a first variant of the device of Fig. 1 wherein some portions have removed to better show others;
- Figs. 23 and 24 show each a front view in median section of the device of Fig. 22 in two operating conditions;
- Fig. 25 shows a front view of a second variant of the device of Fig. 1.

### Best Mode for Carrying Out the Invention

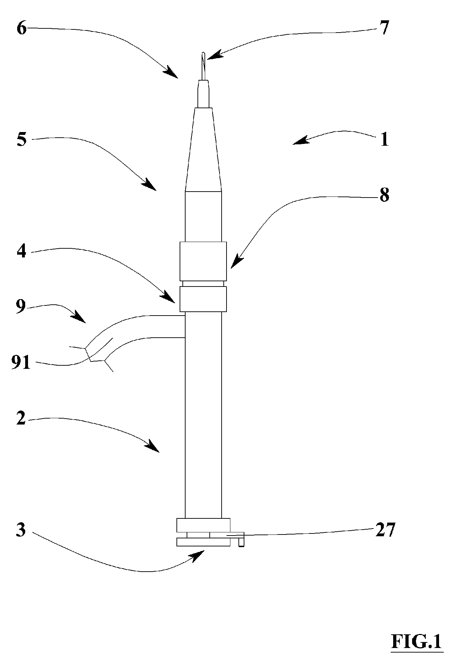

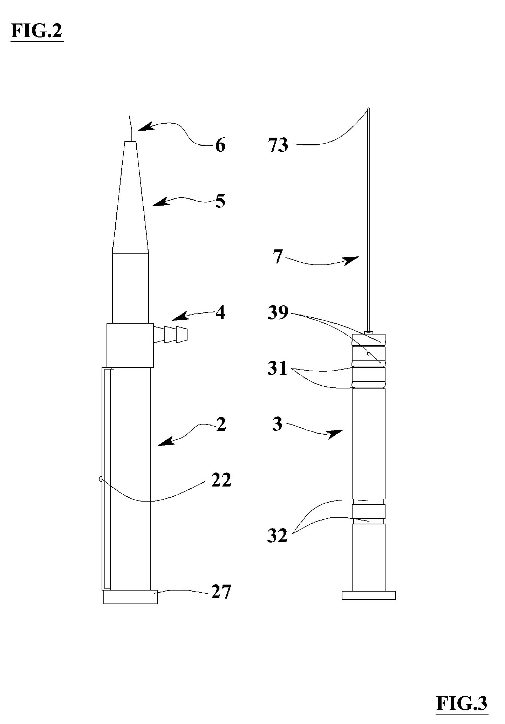

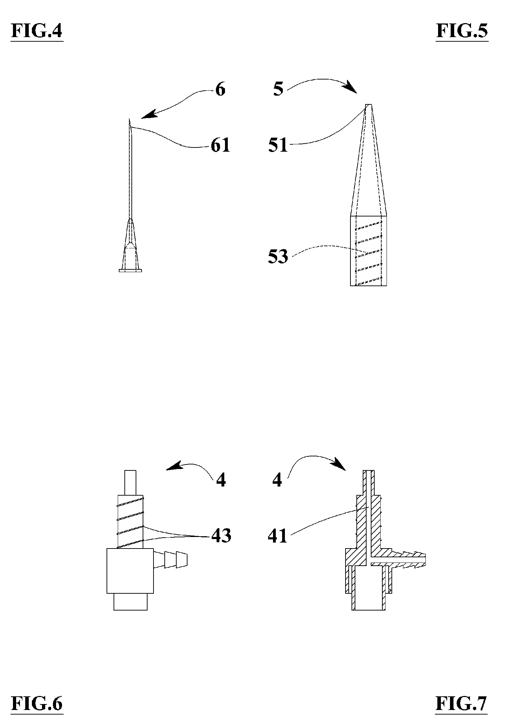

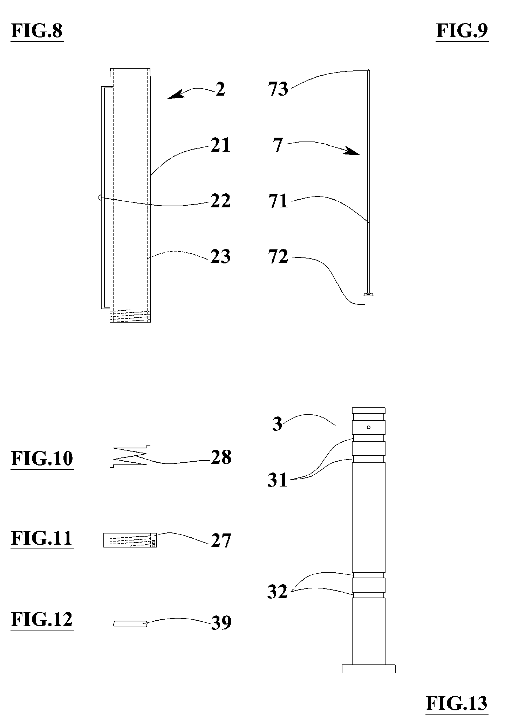

In Figs. 1 to 25 the number 1 indicates a device for tissues sampling and grafting, such as follicular units, in the operations of hair or skin portions transplant.

In the preferred embodiment, shown in Figs. 1 to 21, the device 1 comprises a handle means 2 that is elongated shaped and provided with a first longitudinal through hollow 23.

A coupling means 4 is positioned at an end of the handle means 2 and it is removably coupled to the latter. The coupling means 4 has an inner through duct 41 coaxial with the first through hollow 23 and connected thereto.

A needle means 6 equipped with a lanceolate tip 61 is fixed to the coupling means 4 at the opposite end to which the handle means 2 is connected. The needle means 6 is coaxial with the through duct 41.

The diameter of the needle means 6 is chosen according to the type of operation to be performed. Preferred diameters are usually of 0.6 - 0.8 -1.0 mm for the sampling and grafting operations of single or multiple follicular units, while diameters of 1.2 - 1.5 mm are preferred for sampling and/or grafting for histological, dermal-epidermal, scarring or similar tissue. The person skilled in the art can easily choose the needle means 6 diameter which best suits the specific operation, choosing among the above mentioned or any other diameters, without departing from the scope of the present invention.

Inside the handle means 2 is placed a piston means 3 which slides axially inside the first through hollow 23 and which is equipped with a pin means 7 which is provided with a tip 73 and is fixed to an end of the piston means 3 which remains inside the first through hollow 23.

The pin means 7 slides, together with the piston means 3, inside the through duct 41 and inside the needle means 6. The pin means 7 comprises a needle element 71, having an outer diameter smaller than the inner diameter of the needle means 6, equipped with a coupling element 72 for the removable fixing of the same to the piston means 3.

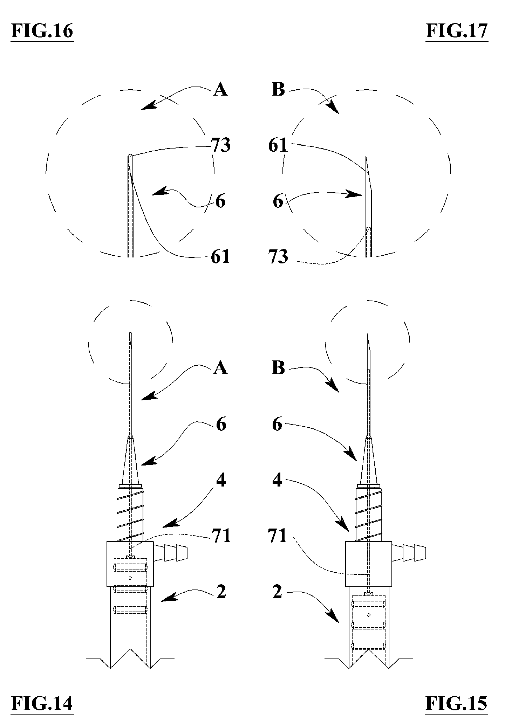

The piston 3 and pin 7 means translate between an external condition A, shown in Figs. 14 and 16, wherein the tip 73 of the pin means 7 projects beyond the lanceolate tip 61, and an internal condition B, shown in Figs. 15 and 17, wherein the tip 73 is retracted inside the needle means 6 leaving free an inner portion of the latter.

The piston means 3 presents some sealing elements 39, such as O-rings, housed in first circumferential grooves 31 that are on the same piston means 3 and parallel to one another. The sealing elements 39 limit the air flow between the handle means 2 and the piston means 3 during the movement of the latter with respect to the handle means, thereby creating an air volume inside the handle means 2 which works as an elastic element opposing to the movement of the piston means 3 with respect to its initial condition wherein it is almost fully inserted in the handle means 2.

The number and shape of the sealing elements 39 are chosen according to the response of the "elastic element" preferred by the operator. A large number of sealing elements 39 provides a slower response, because of the friction generated by the sealing elements 39 opposing to the piston means 3 movement. On the other side, using a limited number of sealing elements 39, up to the limit case of a single sealing element 39, provides a faster response of the "elastic element". In addition to varying the number of sealing elements 39 is also possible to modify the shape of the same and of their respective first circumferential grooves 31 by varying the free space, or clear span, present among the above mentioned elements, since a larger clear span allows a piston means 3 faster actuation.

The device 1 is connected to a suction means 9 via a first suction duct 91 connected to the coupling means 4, and through a flow passage and the duct 41 present in the latter, is in flow communication to the first through hollow 23 and to the needle means 6.

A tip means 5, elongated and tapered near a truncated free end 51 is coupled in a roto-translating way to the coupling means 4 and co-axially surrounds the needle means 6. The tip means 5 moves between a retracted condition C, wherein the lanceolate tip 61 of the needle means 6 protrudes beyond the truncated free end 51 of the tip means 5, and an advanced condition D, wherein the lanceolate tip 61 is completely inside the tip means 5.

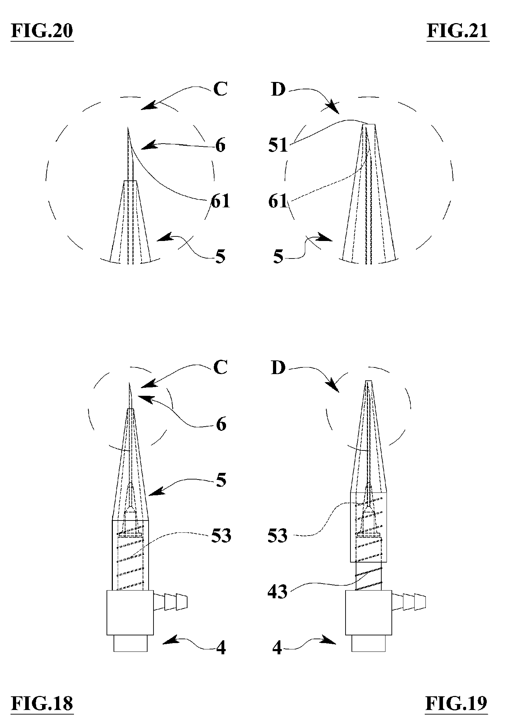

The truncated free end 51 has very thin sidewall, as shown in Figs. 20 and 21, or is sharpened in order to cut the tissue to be sampled.

The roto-translating constraint between the tip means 5 and the coupling means 4 is allowed by the presence of a first threaded element 53, realized inside the tip means 5, to mate a corresponding second threaded element 43 realized on the outer wall of the coupling means 4. The choice of the pitch of the first 53 and second 43 threaded elements is made depending on the desired ratio between the rotation of the tip means 5 with respect to the coupling means 4 and their mutual translation. To obtain a high translation against a limited rotation a coarse-pitch threaded element is used. The person skilled in the art can suitably choose the pitch of the threaded elements without departing from the scope of the present invention.

The handle means 2 comprises also a lock means 27, constituted of an element rotably coupled to the handle means 2, detachably engaging second circumferential grooves 32 present on the piston means 3 to lock the latter 3 in the internal condition B or in intermediate configurations between the external A and internal B ones.

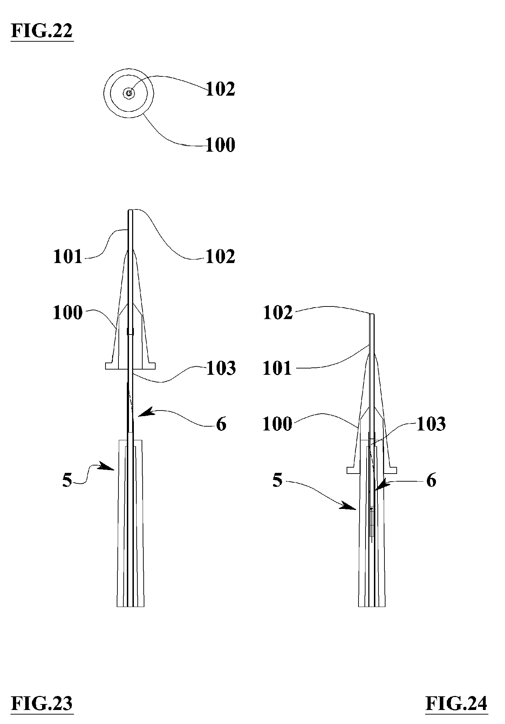

In a first variant of the preferred embodiment, shown in Figs. 22 to 24, the device 1 also comprises a fixing element 100, for example shaped as a Luer Lock or bayonet or quick coupling or truncated cone, which is removably constrainable to the tip means 5.

The fixing element 100 supports a tubular element 101 in coaxial way with respect to the needle means 6 to which it is in flow communication through a tubular portion 103 having an outer diameter smaller than the inner diameter of the needle means 6 so as to be able to translate at least partially inside the latter 6.

The free end 102 of the tubular element 101 is sharpened and cut along a plane orthogonal to the direction along which the tubular element 101 develops, so as to define a "hole saw".

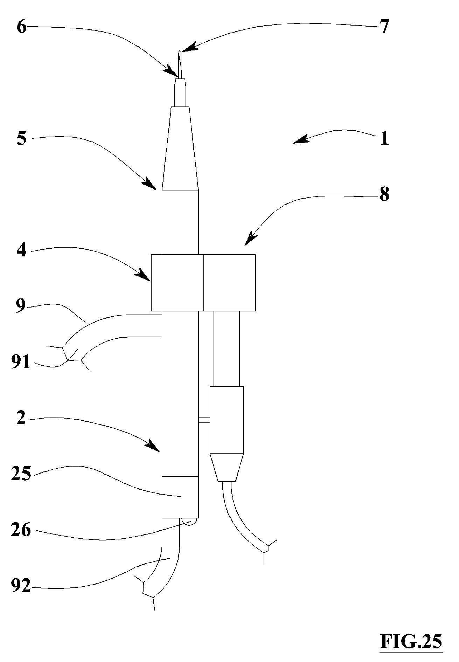

In a second variant of the preferred embodiment, shown in Fig. 25, the device 1 is further equipped with a first actuator means 8 coupled to the coupling means 4 and assigned to rotate the tip means 5 with respect to the coupling means 4. Further in this variant the free end 51 of the tip means 5 can be sharpened. The combination of the first actuator means 8 and the free end 51, that can be sharpened, allows the device to work as a coring drill.

In a third variant of the preferred embodiment, the needle means 6 rotates with respect to the handle means 2 actuated by a second actuator means, such as a torsion spring 28 whose one end is coupled to the handle means 2 and the other end is coupled to the needle means 6.

In a fourth variant of the preferred embodiment, not shown, the device 1 comprises a third actuator means assigned to move the piston means 3 with respect to the handle means 2, and the third actuator means is constituted by a second suction duct 92, connected to the suction means 9 and controlled via a switch element 26, or is constituted by a micro-motor.

In a fifth variant of the preferred embodiment, the device 1 comprises a common switch element 22 coupled to the handle means 2 and assigned to control at the same time the rotation of the needle means 6 and the translation of the piston means 3.

In a sixth variant of the preferred embodiment, the first 8, second 25 and third 28 actuator means are coaxial to the handle means 2, are provided of a hollow driving shaft and are constituted by an actuator chosen among micro-motors, pneumatic, hydraulic or ultrasonic actuators.

In a seventh variant of the preferred embodiment, the device 1 further comprises a display means of the taken tissue sample. This display means is constituted of a transparent portion shaped as a magnifying glass integrated in the tip means 5 near a stop portion wherein the taken tissue sample is retained to visually evaluate the conditions of the sample itself.

Alternatively both the tip means 5 and the needle means 6 are made of transparent materials to allow viewing of the taken tissue sample.

The operation of the device 1 provides that the operator places the device 1 over the portion of tissue to be taken, and in the case of follicular units sampling the device 1 is aligned to the follicular unit same.

In the advanced condition D the device 1 is leaning against the tissue to be taken and the first actuator means is actuated 8 which, by rotating the end 51 of the tip means 5, performs a coring of the affected area around the unit follicular.

Then the piston means 3 is shifted from the external condition A to the internal B one and simultaneously the follicular unit is sucked inside the needle means 6, in the space left by the pin means 7 which is moved integral with the piston means 3.

Then the device 1 is removed from the sampling zone and placed at the grafting area in the retracted condition C, in such a way that the needle means 6 protrudes beyond the free end 51 of the tip means 5.

The end of the needle means 6 is leant on the tissue where the previously taken tissue should be grafted and the needle means 6 rotation is actuated which, via the lanceolate tip 61, creates a circular hole on the affected tissue.

Simultaneously the piston means 3 movement is controlled from the internal condition B to the external A one, and said movement pushes, through the needle means 7, the taken tissue inside the hole created by the lanceolate tip 61.

The simultaneity of operations performed in the grafting step allows to drastically reduce the tissue bleeding, as the graft itself acts as a buffer.

The operation of the first variant of the preferred embodiment provides that the operator constraints the fixing element 100 on the tip means 5, taking care to slide the tubular portion 103 within the needle means 6.

Subsequently, the operator presses the end 102 over the portion of tissue to be sampled and actuates the actuator means which rotates the tip means 5 and consequently also the fixing element 100 and its end 102. In this way the end 102 creates a circular incision around the portion of tissue concerned, which is then sucked inside the needle means 6, in a way similar to the described above operation.

To perform the grafting of the taken tissue, the operator removes the fixing element 100 and operates the steps of the above described operation.

The operation of the seventh variant of the preferred embodiment provides an additional step of visual evaluation of the taken tissue before its grafting by observing the latter through the display means or the transparent portions present on the tip means 5 and the needle means 6.

The main advantage of the present invention is to provide a device for tissue sampling and grafting that reduces the number of operations to be performed also reducing the stress applied to the tissues concerned, to be sampled and grafted, thereby improving their engraftment chances.

Further advantage is to simplify and speed up the sampling and grafting operations by using a single device.

Another advantage is to provide a device suitable to the auto transplant operations of epithelial tissues, removing of scars, epidemic damage due to burns and/or tattoos.

Further advantage is to provide a device that allows to visually evaluate the conditions of the taken tissue sample before grafting the same to ensure a higher grafting success rate.

## Claims

1. Device for tissues sampling and grafting comprising a handle means (2) having an end fixed to a coupling means (4), a needle means (6), whose free end has a lanceolate tip (61) and the other end is fixed to the coupling means (4); wherein
- the handle means (2) has a first longitudinal through hollow (23) communicating with a longitudinal through duct (41) inside the coupling means (4) and communicating with the needle means (6); - a piston means (3) slidable into the first hollow (23) and having a pin means (7), having a tip (73), slidable inside the duct (41) and inside the needle means (6), said piston means (3) and pin means (7) translate between an external condition (A), in which the tip (73) protrudes beyond the lanceolate tip (61), and an internal condition (B), in which the tip (73) is retracted inside the needle means (6); - the piston means (3) has sealing elements (39);- a suction means (9) communicates with the through duct (41); **characterized in that** the suction means (9) communicates with the first hollow (23) and the device further comprises
- a tip means (5) has a truncated free end (51) that is sharpened, is co-axially placed outside the needle means (6) and is coupled in a roto-translating way with respect to the coupling means (4) between a retracted condition (C), in which the lanceolate tip (61) protrudes beyond the tip means (5), and an advanced condition (D), in which the lanceolate tip (61) is completely inside the tip means (5).

2. Device according to claim 1 **characterized in that** the handle means (2) includes a lock means (27) which engages at least one of a set of second circumferential grooves (32) on the piston means (3) to lock this latter at the internal condition (B) or at intermediate condition between the external (A) and the internal (B) conditions.

3. Device according to any of the previous claims **characterized in that** the tip means (5) has at least a first inner threaded element (53) engaging at least a second threaded element (43) carried out outside the coupling means (4) and realizing the roto-translating constraint between the tip (5) and the coupling means (4).

4. Device according to any of the previous claims **characterized by** comprising also a first actuator means (8), constrained to coupling means (4) and rotating the tip means (5) with respect to the coupling means (4).

5. Device according to claim 4 **characterized in that** the sharpened tip means (5) free end (51) cooperates with the first actuator means (8) as a coring drill.

6. Device according to any of the previous claims **characterized by** comprising a second actuator means (28) which rotates the needle means (6) with respect to the handle means (2).

7. Device according to claim 6 **characterized in that** the second actuator means (28) consists of a torsion spring having an end constrained to the handle means (2).

8. Device according to any of the previous claims **characterized by** comprising also a third actuator means (25), constrained to the handle means (2), translating the piston means (3) and consisting of an actuator chosen among a micro-electric, mechanic, hydraulic or ultrasonic actuator, or consisting of a second suction duct (92) connected to the suction means (9).

9. Device according to claim 8 **characterized by** comprising also a common switch element (22) constrained to the handle means (2) and controlling simultaneously the needle means (6) rotation and the piston means (3) translation.

10. Device according to any claims 3-9 **characterized in that** at least one of the first (8), second (25) and third (28) actuator means is coaxial with the handle means (2), is equipped with a hollow motor shaft and is an actuator chosen among micro-motor, pneumatic, hydraulic or ultrasonic actuator.

11. Device according to any of the previous claims **characterized by** comprising displaying means for the taken tissue sample.

12. Device according to any of the previous claims **characterized in that** the tip (5) and needle (6) means are at least partially transparent to allow viewing of the taken tissue sample.

13. Device according to claim 12 **characterized in that** the tip means (5) has at least a transparent portion, shaped as a magnifying glass, near stop portion wherein the taken tissue sample is retained for visually evaluating the conditions of the sample same.

14. Device according to any of the previous claims **characterized in that** a fixing element (100) is detachably constrainable to the tip means (5) and supports a tubular element (101) which is provided with an end (102) that is sharpened on an orthogonal geometric plane with respect to the tubular element (101) and it is coaxial to the needle means (6) with which is in flow communication.

15. Device according to claim 14 **characterized in that** the tubular element (101) is in flow communication with the needle means (6) via a tubular portion (103) that has an outer diameter smaller than the inner diameter of the needle means (6) to be able to at least partially translate inside the latter (6).

## Patentansprüche

1. Vorrichtung zur Gewebeprobennahme und -verpflanzung, umfassend
ein Griffmittel (2), das mit einem Ende an einem Kupplungsmittel (4) befestigt ist,
ein Nadelmittel (6), dessen freies Ende eine lanzenförmige Spitze (61) aufweist und dessen anderes Ende am Kupplungsmittel (4) befestigt ist, wobei
das Griffmittel (2) einen ersten länglichen Durchgangshohlraum (23) aufweist, der mit einer länglichen Durchgangsleitung (41) innerhalb des Kupplungsmittels (4) und mit dem Nadelmittel (6) in Verbindung steht, und
ein in den ersten Hohlraum (23) verschiebbares Kolbenmittel (3) mit einem Stiftmittel (7), das eine Spitze (73) aufweist, die innerhalb der Leitung (41) und innerhalb des Nadelmittels (6) verschiebbar ist, wobei
das Kolbenmittel (3) und das Stiftmittel (7) sich verschieben zwischen einem Außenzustand (A), in dem die Spitze (73) über die lanzenförmigen Spitze (61) herausragt, und einem Innenzustand (B), in dem die Spitze (73) in das Nadelmittel (6) zurückgezogen ist, wobei
das Kolbenmittel Dichtelemente (39) aufweist, sowie
ein Saugmittel (9), das mit der Durchgangsleitung (41) in Verbindung steht, und
die Vorrichtung ist **dadurch gekennzeichnet, dass** das Saugmittel (9) mit dem ersten Hohlraum (23) in Verbindung steht und die Vorrichtung weiterhin umfasst ein Spitzenmittel (5) mit einem kegelstumpfartigen freien Ende (51), das geschärft ist, koaxial außerhalb des Nadelmittels (6) angeordnet ist und gekoppelt ist in einer drehübersetzenden Weise bezogen auf das Kupplungsmittel (4) zwischen einem eingefahrenen Zustand (C), in dem die lanzenförmige Spitze (61) über das Spitzenmittel (5) hervorragt, und einem vorgeschobenen Zustand (D), in dem die lanzenförmige Spitze (61) vollständig im Spitzenmittel (5) angeordnet ist.

2. Vorrichtung gemäß Anspruch 1, **dadurch gekennzeichnet, dass** das Griffmittel (2) ein Sperrmittel (27) umfasst, das mit mindestens einer aus einem Satz von zweiten Umfangsrillen (32) auf dem Kolbenmittel (3) in Eingriff steht, um die Letztere im Innenzustand (B) oder in einem Zwischenzustand zwischen dem Außen- (A) und dem Innenzustand (B) zu verriegeln.

3. Vorrichtung gemäß einem der vorhergehenden Ansprüche, **dadurch gekennzeichnet, dass** das Spitzenmittel (5) mindestens ein erstes inneres Gewindeelement (53) aufweist, das mit mindestens einem zweiten Gewindeelement (43) in Eingriff steht, das ausgeführt ist außerhalb des Kupplungsmittels (4) und den Drehübersetzungszwang zwischen der Spitze (5) und dem Kupplungsmittel (4) realisiert.

4. Vorrichtung gemäß einem der vorhergehenden Ansprüche, **dadurch gekennzeichnet, dass** sie auch ein erstes Betätigungsmittel (8) umfasst, das an das Kupplungsmittel (4) gebunden ist und das Spitzenmittel (5) bezogen auf das Kupplungsmittel (4) dreht.

5. Vorrichtung gemäß Anspruch 4, **dadurch gekennzeichnet, dass** das freie Ende (51) des geschärften Spitzenmittels (5) mit dem ersten Betätigungsmittel (8) als einem Kernbohrer zusammenarbeitet.

6. Vorrichtung gemäß einem der vorhergehenden Ansprüche, **dadurch gekennzeichnet, dass** es ein zweites Betätigungsmittel (28) aufweist, welches das Nadelmittel (6) bezogen auf das Griffmittel (2) dreht.

7. Vorrichtung gemäß Anspruch 6, **dadurch gekennzeichnet, dass** das zweite Betätigungsmittel (28) aus einer Torsionsfeder besteht, die mit einem Ende an das Griffmittel (2) gebunden ist.

8. Vorrichtung gemäß einem der vorhergehenden Ansprüche, **dadurch gekennzeichnet, dass** sie ein drittes Betätigungsmittel (25) aufweist, das an das Griffmittel (2) gebunden ist, das das Kolbenmittel (3) verschiebt und aus einem Aktuator besteht, der ausgewählt ist aus einem mikro-elektrischen, einem mechanischen, einem hydraulischen oder einem Ultraschall-Aktuator, oder das aus einer zweiten Saugleitung (92) besteht, die mit dem Saugmittel (9) verbunden ist.

9. Vorrichtung gemäß Anspruch 8, **dadurch gekennzeichnet, dass** sie ebenso ein gewöhnliches Schaltelement (22) aufweist, das gebunden ist an das Griffmittel (2) und gleichzeitig die Drehung des Nadelmittels (6) und die Translation des Kolbenmittels (3) steuert.

10. Vorrichtung gemäß einem der Ansprüche 3 bis 9, **dadurch gekennzeichnet, dass** mindestens eines des ersten (8), zweiten (25) und dritten Betätigungsmittels (28) koaxial zum Griffmittel (2) verläuft, mit einer hohlen Motorwelle ausgestattet ist und ein Aktuator ist, der ausgewählt ist aus mikro-motor-, pneumatischem, hydraulischem oder Ultraschall-Aktuator.

11. Vorrichtung gemäß einem der vorhergehenden Ansprüche, **dadurch gekennzeichnet, dass** sie eine Anzeige für die entnommene Gewebeprobe aufweist.

12. Vorrichtung gemäß einem der vorhergehenden Ansprüche, **dadurch gekennzeichnet, dass** das Spitzenmittel (5) und das Nadelmittel (6) zumindest teilweise transparent sind, um ein Betrachten der entnommene Gewebeprobe zu gestatten.

13. Vorrichtung gemäß Anspruch 12, **dadurch gekennzeichnet, dass** das Spitzenmittel (5) zumindest einen transparenten Bereich aufweist, der geformt ist als eine Lupe, nahe des Haltebereichs, in dem die entnommene Gewebeprobe zur visuellen Auswertung des Zustands der Probe enthalten ist.

14. Vorrichtung gemäß einem der vorhergehenden Ansprüche, **dadurch gekennzeichnet, dass** ein Befestigungselement (100) lösbar an dem Spitzenmittel (5) befestigbar ist und ein röhrenförmiges Element (101) unterstützt, das mit einem Ende (102) bereitgestellt ist, das auf einer orthogonalen, geometrischen Ebene bezogen auf das röhrenförmige Element (101) geschärft ist und koaxial zum Nadelmittel (6) angeordnet ist, mit dem es sich in Fließverbindung befindet.

15. Vorrichtung gemäß Anspruch 14, **dadurch gekennzeichnet, dass** das röhrenförmige Element (101) sich in der Fließverbindung mit dem Nadelmittel (6) über einen röhrenförmigen Bereich (103) befindet, der einen schmaleren äußeren Durchmesser als der innere Durchmesser der Nadel (6) aufweist, um sich zumindest teilweise in das Letztere (6) zu bewegen.

## Revendications

1. Dispositif d'échantillonnage et de greffe de tissus comprenant un moyen de poignée (2) ayant une extrémité fixée à un moyen de couplage (4), un moyen d'aiguille (6) dont l'extrémité libre a une pointe lancéolée (61) et dont l'autre extrémité est fixée au moyen de couplage (4) ; dans lequel :
- le moyen de poignée (2) a un premier creux traversant longitudinal (23) communiquant avec un conduit traversant longitudinal (41) à l'intérieur du moyen de couplage (4) et communiquant avec le moyen d'aiguille (6) ;
- un moyen de piston (3) apte à coulisser dans le premier creux (23) et ayant un moyen de broche (7), ayant une pointe (73), apte à coulisser à l'intérieur du conduit (41) et à l'intérieur du moyen d'aiguille (6), ledit moyen de piston (3) et ledit moyen de broche (7) se déplaçant en translation entre un état externe (A), dans lequel la pointe (73) fait saillie au-delà de la pointe lancéolée (61), et un état interne (B), dans lequel la pointe (73) est rétractée à l'intérieur du moyen d'aiguille (6) ;
- le moyen de piston (3) a des éléments d'étanchéité (30) ;
- un moyen d'aspiration (9) communique avec le conduit traversant (41) ; **caractérisé par le fait que** le moyen d'aspiration (9) communique avec le premier creux (23) et le dispositif comprend en outre :
- un moyen de pointe (5) qui a une extrémité libre tronquée (51) qui est aiguisée, est placée coaxialement à l'extérieur du moyen d'aiguille (6) et est couplée à rotation-translation par rapport au moyen de couplage (4) entre un état rétracté (C), dans lequel la pointe lancéolée (61) fait saillie au-delà du moyen de pointe (5), et un état avancé (D), dans lequel la pointe lancéolée (61) est entièrement à l'intérieur du moyen de pointe (5).

2. Dispositif selon la revendication 1, **caractérisé par le fait que** le moyen de poignée (2) comprend un moyen de verrou (27) qui engage au moins l'une d'un ensemble de secondes rainures circonférentielles (32) sur le moyen de piston (3) pour verrouiller ce dernier dans l'état interne (B) ou dans un état intermédiaire entre les états externe (A) et interne (B).

3. Dispositif selon l'une quelconque des revendications précédentes, **caractérisé par le fait que** le moyen de pointe (5) a au moins un premier élément fileté interne (53) engageant au moins un second élément fileté (43) porté à l'extérieur du moyen de couplage (4) et réalisant la contrainte de rotation-translation entre le moyen de pointe (5) et le moyen de couplage (4).

4. Dispositif selon l'une quelconque des revendications précédentes, **caractérisé par le fait qu'**il comprend également un premier moyen d'actionneur (8), contraint au moyen de couplage (4) et faisant tourner le moyen de pointe (5) par rapport au moyen de couplage (4).

5. Dispositif selon la revendication 4, **caractérisé par le fait que** l'extrémité libre aiguisée (51) du moyen de pointe (5) coopère avec le premier moyen d'actionneur (8) comme un foret de carottage.

6. Dispositif selon l'une quelconque des revendications précédentes, **caractérisé par le fait qu'**il comprend un deuxième moyen d'actionneur (28) qui fait tourner le moyen d'aiguille (6) par rapport au moyen de poignée (2).

7. Dispositif selon la revendication 6, **caractérisé par le fait que** le deuxième moyen d'actionneur (28) consiste en un ressort de torsion ayant une extrémité contrainte au moyen de poignée (2).

8. Dispositif selon l'une quelconque des revendications précédentes, **caractérisé par le fait qu'**il comprend également un troisième moyen d'actionneur (25), contraint au moyen de poignée (2), déplaçant en translation le moyen de piston (3) et consistant en un actionneur choisi parmi un actionneur micro-électuque, mécanique, hydraulique ou ultrasonore, ou consistant en un second conduit d'aspiration (92) relié au moyen d'aspiration (9).

9. Dispositif selon la revendication 8, **caractérisé par le fait qu'**il comprend également un élément commutateur commun (22) contraint au moyen de poignée (2) et commandant simultanément la rotation du moyen d'aiguille (6) et la translation du moyen de piston (3).

10. Dispositif selon l'une quelconque des revendications 3 à 9, **caractérisé par le fait qu'**au moins l'un des premier (8), deuxième (25) et troisième (28) moyens d'actionneur est coaxial avec le moyen de poignée (2), est équipé d'un arbre de moteur creux et est un actionneur choisi parmi un actionneur micromoteur, pneumatique, hydraulique ou ultrasonore.

11. Dispositif selon l'une quelconque des revendications précédentes, **caractérisé par le fait qu'**il comprend des moyens d'affichage pour l'échantillon de tissu prélevé.

12. Dispositif selon l'une quelconque des revendications précédentes, **caractérisé par le fait que** les moyens de pointe (5) et d'aiguille (6) sont au moins partiellement transparents pour autoriser la visualisation de l'échantillon de tissu prélevé.

13. Dispositif selon la revendication 12, **caractérisé par le fait que** le moyen de pointe (5) a au moins une partie transparente, sous la forme d'une loupe, près d'une partie d'arrêt dans laquelle l'échantillon de tissu prélevé est retenu pour évaluer visuellement l'état de l'échantillon.

14. Dispositif selon l'une quelconque des revendications précédentes, **caractérisé par le fait qu'**un élément de fixation (100) est apte à être contraint de façon détachable au moyen de pointe (5) et supporte un élément tubulaire (101) qui est muni d'une extrémité (102) qui est aiguisée sur un plan géométrique orthogonal par rapport à l'élément tubulaire (101), et il est coaxial au moyen d'aiguille (6) avec lequel il est en communication fluidique.

15. Dispositif selon la revendication 14, **caractérisé par le fait que** l'élément tubulaire (101) est en communication fluidique avec le moyen d'aiguille (6) par l'intermédiaire d'une partie tubulaire (103) qui a un diamètre externe plus petit que le diamètre interne du moyen d'aiguille (6) pour être apte à se déplacer partiellement en translation à l'intéueur de ce dernier (6).
